(19) **Europäisches Patentamt**
**European Patent Office**
**Office européen des brevets**

(11) **EP 2 701 578 B1**

(12) **EUROPEAN PATENT SPECIFICATION**

(45) Date of publication and mention
of the grant of the patent:
**16.06.2021 Bulletin 2021/24**

(51) Int Cl.:
***A61B 5/00*** *(2006.01)*     ***A61B 3/113*** *(2006.01)*

(21) Application number: **12739478.1**

(86) International application number:
**PCT/US2012/022959**

(22) Date of filing: **27.01.2012**

(87) International publication number:
**WO 2012/103470 (02.08.2012 Gazette 2012/31)**

(54) **METHOD OF DETECTING NEUROLOGICAL DISEASE**

VERFAHREN ZUM NACHWEIS EINER NEUROLOGISCHEN ERKRANKUNG

PROCÉDÉ DE DÉTECTION DE MALADIE NEUROLOGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.01.2011 US 201161437138 P**

(43) Date of publication of application:
**05.03.2014 Bulletin 2014/10**

(73) Proprietor: **Dignity Health**
**Phoenix, AZ 85012 (US)**

(72) Inventors:
 • **MARTINEZ-CONDE, Susana**
 **Orinda, CA 94563 (US)**
 • **MACKNIK, Stephen, L.**
 **Brooklyn, NY 11201 (US)**
 • **TRONCOSO, Xoana**
 **Phoenix, AZ 85013 (US)**

 • **OTERO-MILLAN, Jorge**
 **Phoenix, AZ 85014 (US)**

(74) Representative: **Croston, David**
**Withers & Rogers LLP**
**4 More London Riverside**
**London**
**SE1 2AU (GB)**

(56) References cited:
 **WO-A1-2009/059167**    **WO-A2-2010/042557**
 **US-A1- 2003 028 081**    **US-A1- 2010 191 156**
 **US-A1- 2010 277 693**

 • **ATHENA L. CHEN ET AL: "The Disturbance of Gaze in Progressive Supranuclear Palsy: Implications for Pathogenesis", FRONTIERS IN NEUROLOGY, vol. 1, 147, 3 December 2010 (2010-12-03), XP055358416, DOI: 10.3389/fneur.2010.00147**
 • **None**

**Description**

Field of the Invention

The field of the invention relates to using eye movements as a way to provide differential diagnosis of progressive supranuclear palsy from Parkinson's disease.

Background of the Invention

The eye movements of people with neurological disease differ significantly from those of healthy people. In addition, eye movements among people with seemingly similar but different neurological diseases can also be different from each other. Because of the importance of accurate diagnosis of neurological diseases, a need exists for better methods of evaluating such differences.

The eyes do not stay perfectly still during visual fixation. Fixational eye movements and saccadic intrusions continuously change the position of the gaze.

Microsaccades are rapid, small-magnitude involuntary saccades that occur several times each second during fixation; microsaccades counteract visual fading and generate strong neural transients in the early visual system. Microsaccades may also drive perceptual flips in binocular rivalry. Microsaccade rates and directions are moreover modulated by attention, and thus generate rich spatio-temporal dynamics. Further, fixational eye movements as a whole enhance fine spatial acuity.

The most common type of saccadic intrusion is referred to as a square wave jerk (SWJ). SWJs are characterized by one small horizontal saccadic movement that moves the eye away from the fixation target, followed by a corrective saccade towards the target shortly thereafter. SWJs are prevalent in some neurological diseases such as progressive supranuclear palsy (PSP). However, they are also common in normal healthy subjects and in patients with Parkinson's disease (PD).

Chen, A.L., et al (2010) Frontiers in Neurology; 1 (147): 1-19 describe the use of eye movements for investigating the pathogenesis of PSP, and the disturbance of gaze as a clinical feature of the disease.

Patients with PSP and those with early stages of PD often appear to present similarly. It would be beneficial to be able to differentially diagnose one disease from the other in a non-invasive manner. The following disclosure provides one such differential diagnostic method.

Summary

The invention is defined in the appended claims.

A method and apparatus are disclosed for detecting the eye movements of a patient to provide a differential diagnosis of progressive supranuclear palsy (PSP) from Parkinson's disease (PD) in that patient. The method includes the step of identifying a plurality of at least partially repetitive eye movements of a patient to be diagnosed with one or the other of PSP or PD that over time define square wave jerks within a sequence of eye movements. Each square wave jerk of the plurality of square wave jerks is defined by a first horizontal saccadic movement that moves the eye away from a fixation target that is followed by a corrective saccadic movement towards the target shortly thereafter. A vertical component associated with the plurality of square wave jerks is measured. The vertical component is compared with a predetermined threshold value, such as the mean of control data $\pm 1$ standard deviation, and the presence of PSP or PD is identified based upon the comparison of the vertical component with the threshold value whereby a vertical component statistically different (mean $\pm 1$ standard deviation) from that of a normal subject or that PD patients identifies the patient as having PSP, whereas a vertical component that is (a) not statistically different (mean $\pm 1$ standard deviation) from that of normal healthy subjects or PD patients or (b) is statistically different (mean $\pm 1$ standard deviation) from that of PSP patients, identifies the patient as having PD.

Also contemplated is another method for providing a differential diagnosis of progressive supranuclear palsy (PSP) from Parkinson's disease (PD) in that patient. Here, the method includes the step of identifying a plurality of at least partially repetitive eye movements of a patient to be diagnosed with one or the other of PSP or PD that over time define square wave jerks within a sequence of eye movements. Each square wave jerk of the plurality of square wave jerks is defined by a first horizontal saccadic movement that moves the eye away from a fixation target that is followed by a corrective saccadic movement towards the target shortly thereafter. The saccade rate [number of saccades per unit time, e.g., number per second (N/s)] is determined and that rate is compared with a predetermined threshold value, such as the mean of control data $\pm 1$ standard deviation, and the presence of PSP or PD is identified based upon the comparison of the saccade rate with the threshold value whereby a saccade rate statistically different (mean $\pm 1$ standard deviation) from that of normal subjects or from that PD patients identifies the patient as having PSP, whereas a saccade rate that is statistically different (mean $\pm 1$ standard deviation) from that of normal subjects or PSP patients identifies the patient as having PD.

As used herein, the word "subject" with or without modifiers such as "healthy" and "normal" refers to a person free from apparent symptoms of PSP or PD, data from whom are used as control values. A group of "subjects" is sometimes referred to herein as a "healthy population".

Brief Description of the Drawings

Fig. 1 is a block diagram of a system for detecting and characterizing square wave jerks in the eye movements of a subject to diagnose neurological disease, shown generally in accordance with an illustrated embodiment of the invention;
Fig. 2 is a number of graphs of saccades of patients and healthy subjects;
Fig. 3 is a flow chart of steps that may be followed by the system of Fig. 1;
Fig. 4 graphically depicts steps in identifying square wave jerks that may be followed by the system of Fig. 1;
Fig. 5 compares statistics of patients and healthy subjects that can be provided by the system of Fig. 1;
Fig. 6 is a graph of saccade magnitude (abscissa) versus saccade speed (ordinate) for PD patients (**o**) and PSP patients (**x**); and
Fig. 7 in two panels, as Fig. 7A and Fig. 7B, provides two graphs that show saccade data from PSP patients (**x**), PD patients (**o**) and normal healthy subjects ($\Delta$), and in which ovals surrounding the data points indicate the region of the mean $\pm$ 1 standard deviation.

Detailed Description of an Illustrated Embodiment

A process is described herein that automatically identifies SWJs in the eye movements of a person, during visual fixation of a small target. The results show that SWJs are common in both PSP patients and normal healthy subjects. Other results show that SWJs are also common in Parkinson's disease (PD) patients. However, several SWJ parameters (e.g., SWJ rates, magnitudes, percentage of small saccades that are part of SWJs, average inter- saccadic intervals for the SWJs, saccadic rates, saccadic peak velocities within SWJs, standard deviation of the direction difference between pairs of saccades in the SWJs, standard deviation of the difference between the horizontal and the direction of the saccades in the SWJs) have been found to be different in the PSP group.

The vertical components of SWJs of PD and healthy subjects are similar; i.e., not statistically significantly different (mean $\pm 1$ standard deviation). On the other hand, the saccade rate between PD patients and normal healthy subjects is just different enough that the two can be distinguished. That is, the saccade rates of PD patients and normal healthy subjects are statistically significantly different (mean $\pm 1$ standard deviation).

Thus, the objective characterization of SWJs can provide a powerful tool in the differential diagnosis of oculomotor diseases such as PSP and PD.

Although people spend about 80% of their waking lives fixating their gaze, the contribution of impaired fixational eye movements to vision loss has been overlooked as a potential clinical malady. This gap in knowledge has prevented the field from developing new treatments and diagnostics to ameliorate visual deficits due to impaired fixational eye movements.

In general, a healthy subject or a patient will fixate on a target while his/her eye movements are recorded with an eye tracking system. Any eye tracking system available can be used for this purpose: video tracking, scleral search coil, etc. The temporal and spatial resolution of the eye tracking systems is ideally high enough to allow the detection of small saccades during fixation. A sampling rate of 500 Hz or higher is recommended, although small saccades can nevertheless be detected with lower rates at the expense of non-optimal performance.

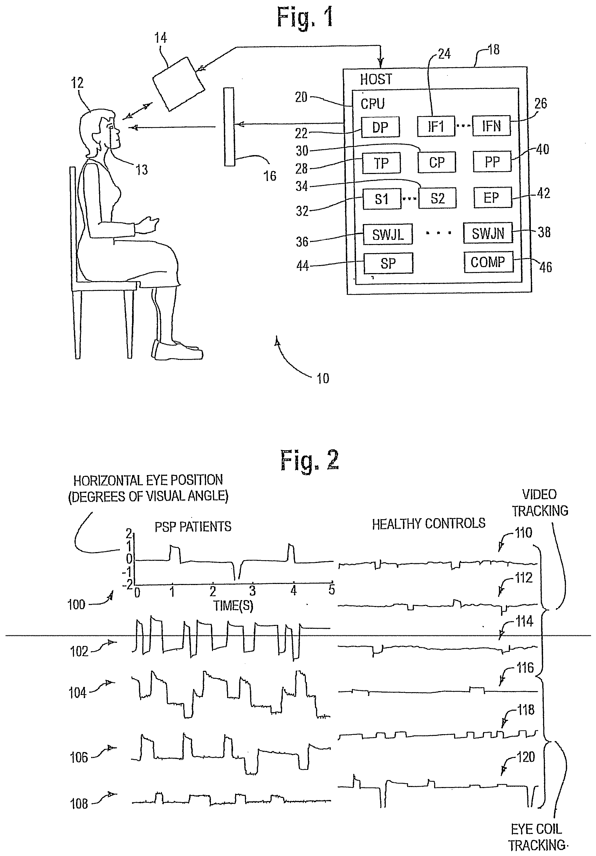

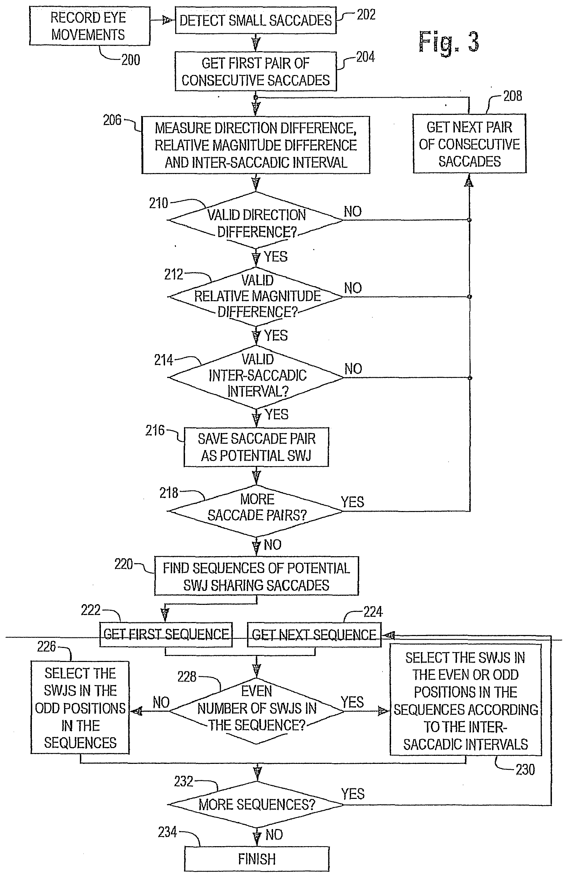

Fig. 1 shows an example of an eye tracking system 10 for detecting eye movement under an illustrated embodiment of the invention. Fig. 3 is a flow chart of steps that can be followed by the system 10. Included within the system 10 can be an eye tracking device 14, such as the EyeLink II by SR Research (sr-research.com/fixed_tech_spec.php) or other equivalent eye tracking systems such as the IVIEW™ HI-SPEED 1250 tracking system by SensoMotoroic Instruments (smivision.com/en/eye-gaze-tracking-systems/products/iview-x-hi-speed.html).

Also included within the system 10 can be a display 16 and host 18. The host 18 includes a central processing unit (CPU) 20 embodied as hardware and a number of associated processors (described below), that can also be embodied as hardware. In this case, the processors can each be defined by a respective hardware processor executing one or more programs loaded from a non-transitory computer readable medium (memory).

The objective of the data collection of the system 10 is to automatically and objectively detect square wave jerks (SWJs) present in the eye movement trace. SWJs are characterized by one small horizontal saccade that moves the eye away from the fixation target, followed by a corrective saccade towards the target shortly thereafter.

Fig. 2 provides examples of eye movement recordings for PSP patients and healthy subjects. A first graph 100 shows an example of horizontal eye position in degrees of visual angle versus time. Graphs 102, 104, 106, 108 provide other examples of eye position versus time for a group of PSP patients, whereas graphs 110, 112, 114, 116, 118 and 120 provide examples of eye position for healthy test subjects.

A display processor 22 within a controller 20 of the system 10 presents the fixation target to a subject 12 on the display 16. As the subject 12 fixates in the target on the display 16, the eye tracking device 14 detects and records 200 the position and movement of the eyes 13 of the subject 12. A tracking processor 28 within the host 18 can receive the position of the eyes 13 and store it for later transfer to a saccade processor 30.

The saccade processor 30 can receive the eye position measurements, can detect 202 substantially all the consecutive pairs of saccades (up to a certain maximum magnitude, for instance, 5 degrees). Any method to detect small saccades can be used by the saccade processor 30. Two main algorithms have been used in the literature: the Martinez-Conde and Macknik algorithm [Martinez-Conde, Macknik, Hubei (2000) Nature Neuroscience 3:251-258] and the Engbert algorithm [Engbert, Kliegl (2003) Vision Res 43:1035- 1045].

The first step of the Martinez-Conde and Macknik process that can be used by the saccade processor 30 is the differentiation of the data (horizontal and vertical position), so that each element represents the instantaneous velocity of the eye in horizontal and vertical space, then data can then be smoothed with a 31 milliseconds (ms) wide unweighted boxcar filter to reduce noise. Then, the direction and size of the motion between each two samples is calculated. The size of the motion represents the velocity of movement in polar coordinates and the direction is differentiated to obtain the rate-of-turn indicator. The saccade processor 30 determines that the eye is moving when the polar velocity is more than 3° per second (s) and the rate-of-turn is smaller than 15°. Finally, only detected eye movements of more than 3 arc minutes (arcmin) and less than 2° are considered saccades.

Under the Engbert process, the saccade processor 30 can first transform the time series of eye positions into velocities in accordance with the equation

$$\vec{v}_n = \frac{\vec{x}_{n+2} + \vec{x}_{n+1} - \vec{x}_{n-1} - \vec{x}_{n-2}}{6\Delta t},$$

that represents a moving average of velocities over 5 data samples in order to suppress noise. As a consequence of the random orientations of the velocity vectors during fixation, the resulting mean value of noise is effectively zero. A multiple of the standard deviation of the velocity distribution is used as the detection threshold. Detection thresholds are computed independently for horizontal and vertical components and separately for each trial, relative to the noise level.

Typical values for the threshold are 4, 5 or 6 times the standard deviation of the velocity. Therefore, the process used by the saccade processor 30 is robust with respect to different noise levels between different trials and subjects. Additionally, minimum saccade duration of 8 or 12 ms is required to further reduce noise. Finally, only binocular saccades are used, that is, saccades with at least 1 sample of overlap between the two eyes.

The principal advantage of the Engbert algorithm is that it adapts to the level of noise of the data. However, although this improves its performance in noisy situations it can produce non-optimal results in low noise conditions where the Martinez-Conde and Macknik algorithm behaves better.

As the saccades 32, 34 are identified (or after), a pairing processor 40 can determine and combine consecutive pairs of associated saccades 32, 34 into potential SWJs 36, 38. The pairing processor I 40 can get a first pair of consecutive saccades 204 and measure a direction difference, a relative magnitude difference and an inter-saccade difference 206. The pairing processor 40 can use three criteria 210, 212, 214 to determine whether a pair of saccades 32, 34 is a SWJ 36, 38. If a pair of saccades 32, 34 does not meet each of the three criteria, then the pair can be discarded.

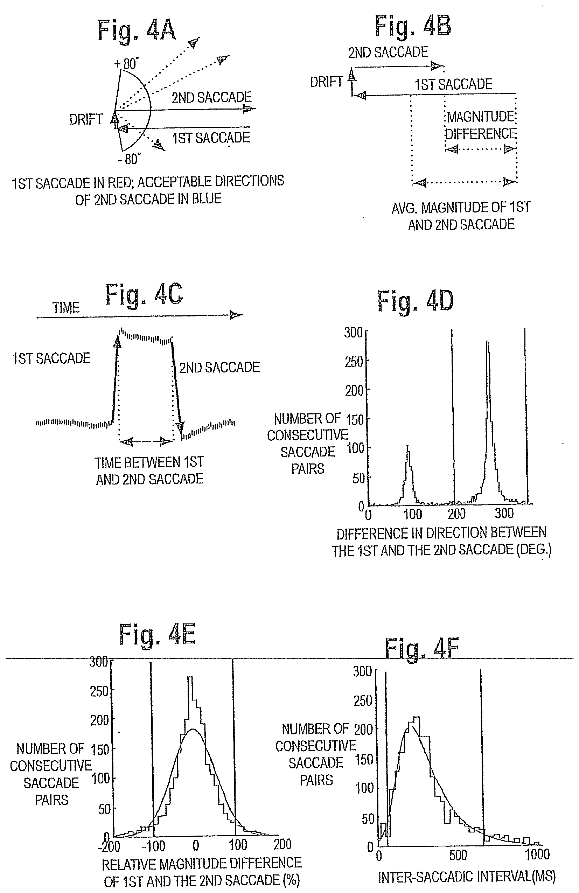

The First criterion requires that the two consecutive saccades 32, 34 should have (loosely) opposite directions. In a perfect SWJ this difference would be exactly 180°. Allowing for some variability, a pair of saccades meets this criterion 210 if the direction difference is in the range 180° + 80°. (See FIGs. 4A and 4D).

The second criterion 212 is that the two consecutive saccades 32, 34 should have similar magnitudes as shown in FIGs. 4B and 4E. A disimilarity index can be objectively calculated as the magnitude difference between the 1st and the 2nd saccade divided by the average magnitude of both saccades (expressed in percent terms) by the following equation,

$$\frac{magnitude\ of\ 1^{st}\ saccade - magnitude\ of\ 2^{nd}\ saccade}{average\ magnitude\ of\ 1^{st}\ and\ 2^{nd}\ saccade} \times 100$$

An ideal SWJ (where the two saccades have equal magnitudes) would have an index of 0%. A pair of saccades is considered a SWJ if the index is in the range $\pm 100\%$.

The third criterion 214 can require that the two consecutive saccades should have Inter-Saccadic Interval (ISI) (between the end of the 1st saccade and the beginning of the 2nd saccade) in the range 70ms - 650ms. (See FIGs. 4C .and 4F).

Once the saccade processor 30 has processed a saccade pair, the processor 30 can determine if there are any more saccade pairs 218. If so, then the saccade processor 30 retrieves the next pair 208 of the sequence and the process repeats.

The specific numeric values for the three different criteria were optimized based on a data from a set of PSP patients (see FIGs. 4D, 4E, 4F). The system 10 can also be used with other criteria values where the other criteria values show a better performance.

As a last step, an elimination processor 42 can locate sequences of potential SWJs sharing saccades 220 and eliminate the SWJs 36, 38 that share saccades 32, 34. The result of the previous step is a sequence of pairs of saccades that meet the initially defined SWJ criteria. However, it is possible in some cases that these pairs are linked by a shared saccade. To solve this problem and have saccades that are only part of a unique SWJ, the following rule can be used: if the number of SWJs linked by shared saccades is odd, then the SWJs in even positions in the sequence of SWJs are discarded.
That is one way to ensure that all the saccades are part of only one SWJ.

If the number is even, then it is impossible to achieve this result and at least one saccade will not be part of any SWJ. In this case the odd or even SWJs can be discarded depending upon which choice provides a shorter average inter-saccadic interval.

As such, the elimination processor 42 can retrieve a first sequence 222 of potential SWJs and determine the number of potential SWJs that share a saccade. If the number of SWJs with shared saccades is even 228, then the elimination processor 42 selects the SWJs in the even or odd positions in the sequence according to the inter-saccadic intervals 230. If not, then the elimination processor 42 selects the SWJs in the odd positions of the sequence 226. If there are any more sequences 232, the process repeats. If not, then the elimination process ends 234.

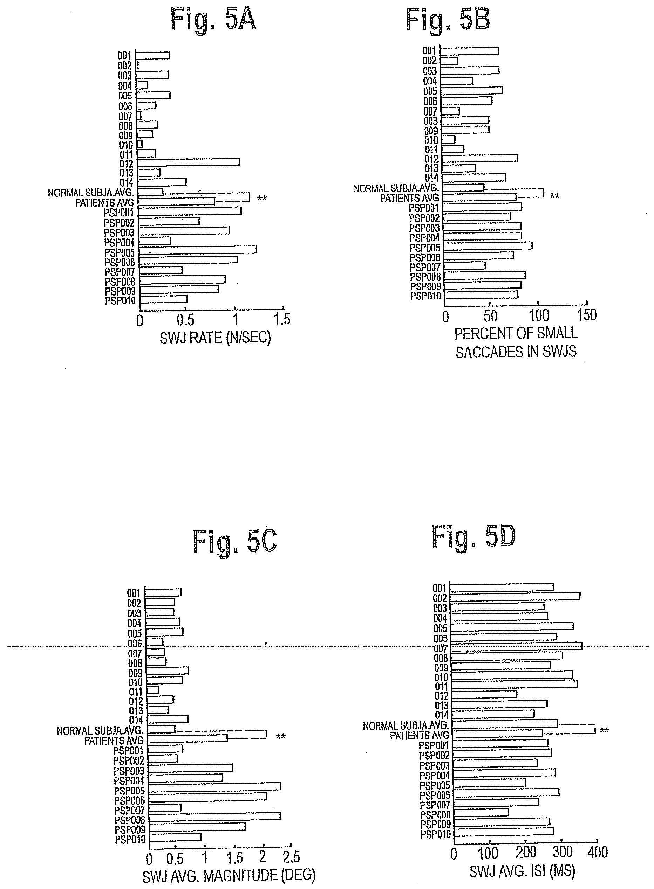

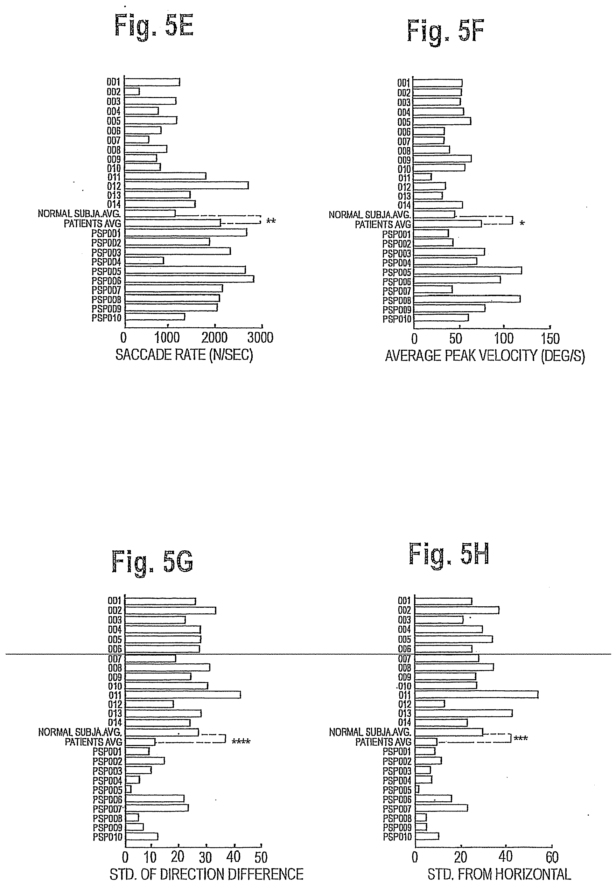

Following identification of SWJs 36, 38, that meet the appropriate criteria, the remaining SWJs 36, 38 can be transferred to a statistics processor 44. Within the statistics processor 44, the SWJs 36, 38 of PSP patients can be compared with healthy subjects. Fig. 5 provides SWJ parameter comparison between a population of healthy subjects and a population of PSP patients.

The system 10 can be used to automatically compute several SWJ parameters that can help to determine whether a person is healthy (a subject) or has certain neurological diseases. In all the panels of Fig. 5, the upper rows (labeled "001" through "014") correspond to respective healthy subjects and the lower rows (labeled "PSP001" through "PSP010") correspond to respective PSP patients. The horizontal bar (labeled "Normal subjs.avg.") is the average of the healthy subjects' population and the horizontal bar (labeled "Patients.avg.") is the average of the PSP patients' population respectively.

The parameters represented in each panel of Fig. 5 are (from left to right and top to bottom): (A)Number of SWJ per second; (B) Percentage of small saccades that are part of SWJs; (C) Average magnitude of the saccades that are part of SWJs; (D) Average inter-saccadic interval for the SWJs; (E) Number of saccades per second; (F) Average peak velocity of the saccades in SWJs; (G) Standard deviation of the direction difference and (H) Standard deviation of the difference between the horizontal and the direction of the saccades in the SWJs. All the parameters (with the exception of the inter-saccadic interval) are significantly different between the two populations (two-tailed t-test).

Any of a number of the statistics of Fig. 5 can be the basis of a screening test for detecting PSP. For example, the standard deviation of direction difference in Fig. 5 (G) shows a greater than a two to one difference between the PSP patients and normal subjects. In this case, the standard deviation of direction difference of a subject 12 tested with the system 10 can be compared within a comparator 46 with the standard deviation of direction difference of a normal subjects to detect PSP. Other statistics of Fig. 5 can be used in a similar manner.

In another embodiment of the invention, differences in SWJ characteristics between normal patients (healthy subjects) and patients with Progressive Supranuclear Palsy (PSP) can also be used to identify patients with Parkinson's disease (PD). In their early stages, these two afflictions cannot be definitively or differentially diagnosed under previously known methods because their symptoms are so similar. It is critical nevertheless to differentiate between them as early as possible as their neurological bases and treatment regimens are quite different. It has been found that the characteristics of SWJs in these two diseases are different, and that the measurement of SWJ characteristics in these two patient populations therefore provide as a sensitive method to differentially diagnose these diseases earlier than other tests.

It is also believed that the measurement of SWJ characteristics similarly serve as a sensitive test for other neurological disorders as well. Examples include such afflictions as stroke, Friedrich's ataxia, cerebellar disease, multiple sclerosis, cerebral lesions, strabismus, and nystagmus.

It has been found that the method described herein accurately differentially diagnoses PSP from PD, in part, because PD patients are more like normal patients for the types of eye movements used in the diagnosis. This is a major advance because there is no previous method to accurately and non-invasively differentially diagnose PSP from PD.

PSP is a much more debilitating disease than PD, although at the early stages they appear similar. Because of the similar symptoms, patients with PSP are often misdiagnosed as having PD and are given L- DOPA or other PD drugs in levels appropriate for PD, but way too low for PSP patients. As a result, PSP patients misdiagnosed with PD typically suffer much more than they otherwise would, had they been accurately diagnosed. The method described below demonstrates that accurate diagnosis of PSP can be made from a simple non-invasive eye movement analysis.

In this regard, it has been found that PSP is distinguishable from Parkinson's disease because the vertical component of SWJ's is smaller for PSP patients than for Parkinson's patients. This effect exists between PSP patients and healthy people (subjects), as well.

Detection in this regard can include an eye movement processing apparatus (including one or more of device 14 and special purpose processors 20, 30, 40) that detects SWJs as discussed above. One or more programmed processors, such as those shown in Fig. 1 (e.g., processors 42, 44), can then analyze a vertical component of each of the SWJs.

In this regard, the vertical component can be manifested as a tilting of the horizontal saccades by a few degrees. As above, a standard deviation of directional difference can be determined in the vertical component for the deviation away from the fixation target versus the corrective saccade towards the target. This can be compared with a normal subject and/or with normal subjects as a basis for determining a set of threshold values for PSP and Parkinson's disease.

In addition, the number of vertical deviations per time period as well the magnitude of the vertical deviation can be collected. The velocity of the vertical deviation can be measured.

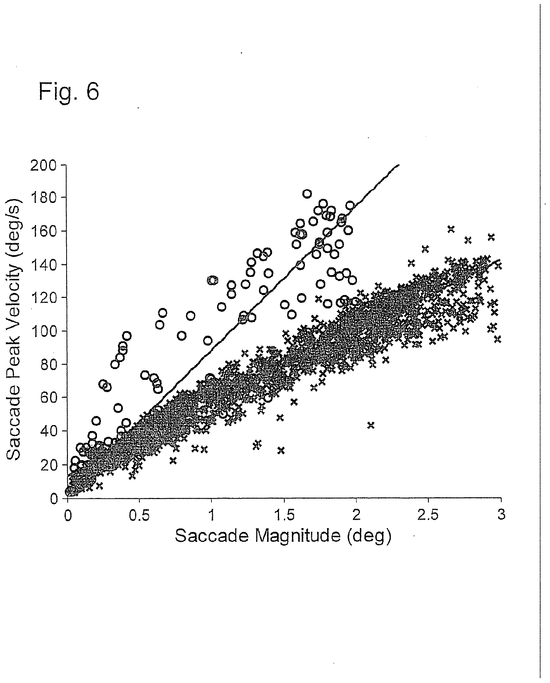

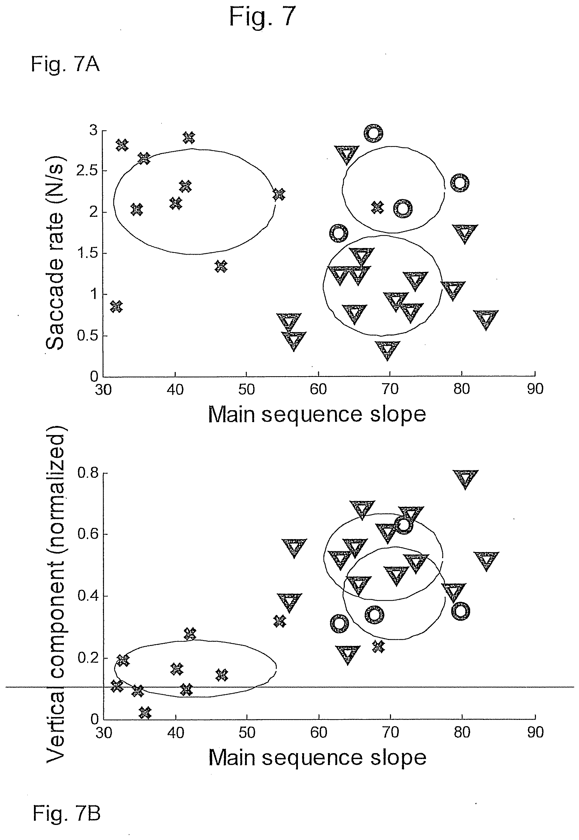

The data points of Fig. 6 illustrate individual saccades. It is seen the PD patients have faster saccades than PSP patients. These data provide the "main sequence slope" utilized in Fig. 7 in which saccade rate is plotted vs. main sequence slope in Fig. 7A and the normalized saccade vertical component is plotted vs. main sequence slope in Fig. 7B.

The data used in FIGs. 6 and 7 were subjected to statistical analysis. The significance testing statistics in the table below were calculated using ANOVA and corrected for multiple comparisons using Tukey's Honest Significant Difference. As is seen, differences between control (normal healthy) patients and PSP patients were highly significant, as were differences between PSP and PD patients for vertical component and main sequence slope, whereas there was little difference in saccade rate between PSP and PD patients. There were also not significant differences between control and PD patients in vertical component and main sequence slope.

Multiple Comparisons Between Groups (adjusted p-values)

|  | Vertical component | Main sequence slope | Saccade rate |
|---|---|---|---|
| Control-PSP | 0.0000025 | 0.0000022 | 0.0071293 |
| Control-PD | 0.4250289 | 0.9916481 | 0.0320478 |
| PSP-PD | 0.0233523 | 0.0002209 | 0.9880972 |

The algorithm used successfully characterized patients in each group (PSP, PD or control) based on three dynamical eye movement parameters in fixation saccades: vertical component, rate and velocity. PSP patients are distinguished from controls based on lack of vertical component, slower velocity or higher rate. PSP and PD patients are distinguished from each other because PSP patients have slower and more horizontal saccades. PD patients and controls (normal healthy subjects) can be distinguished because PDs have higher saccade rates.

The collected vertical data can be compared with that of PSP patients as well as that of normal subjects. A first set of threshold values can be used to identify patients with Parkinson's disease. In a similar manner, a second set of threshold values can be used to identify patients with PSP. Other threshold values can be used to identify patients with stroke, Friedrich's ataxia, cerebellar disease, multiple sclerosis, cerebral lesions, strabismus, and nystagmus.

A specific embodiment of method and apparatus for detecting and characterizing square wave jerks in the eye movements of a subject, which can provide a powerful tool in the differential diagnosis of oculomotor and neurological disease, has been described for the purpose of illustrating the manner in which the invention is made and used. The method disclosed herein has extended the previously filed method of application Serial No. 12/740,008 to include the capability of distinguishing PSP patients and control subjects from each other as well as from and patients affected with Parkinson's Disease (PD).

It should be understood that the implementation of other variations and modifications of the invention and its various aspects will be apparent to one skilled in the art, and that the invention is not limited by the specific embodiments described. The use of the article "a" or "an" is intended to include one or more.

**Claims**

1. A method for controlling an apparatus that differentiates between a patient with progressive supranuclear palsy (PSP) and a patient with Parkinson's disease (PD), the method comprises the steps of:

   a) controlling a processor to determine a saccade rate of data previously obtained from a patient and a vertical component of the data previously obtained from a patient associated with a plurality of square wave jerks;
   b) controlling a processor to compare the saccade rate with a threshold value and the vertical component with a threshold value;
   c) controlling a processor to identify the presence of PSP or PD in the patient based upon the comparison of the saccade rate and the vertical components with the threshold values, whereby a saccade rate statistically different from the threshold value and a vertical component statistically different from the threshold value identifies the patient to be diagnosed as having PSP, whereas a saccade rate statistically different from the threshold value and a vertical component not statistically different from the threshold value identifies the patient as having PD, wherein the data previously obtained from the patient comprises a recording of a plurality of at least partially repetitive eye movements during gaze fixation that over time define square wave jerks, each square wave jerk being defined by a first horizontal saccadic movement that moves the eye away from a fixation target that is followed by a corrective saccadic movement towards the target shortly thereafter.

2. The method as in claim 1 wherein said sequence of saccadic movements was previously acquired using a processor.

3. The method as in claim 2 further comprising controlling a processor to identify pairs of consecutive saccadic movements of the sequence.

4. The method as in claim 3 further comprising controlling a processor to determine whether each saccadic movement of each identified pair is opposite the direction of the other saccadic movement and, if not, then discarding the pair.

5. The method as in claim 4 further comprising controlling a processor to determine whether a magnitude of each saccadic movement of each identified pair is comparable and, if not, then discarding the pair.

6. The method as in claim 5 further comprising controlling a processor to determine whether the pair of saccadic movements of each identified pair are temporally related by a predetermined time period and, if not, then discarding the pair.

7. The method as in claim 6 wherein any remaining pairs of saccadic movements were previously acquired using a processor as square wave jerks.

8. The method as in claim 7 further comprising controlling a processor to compare a set of parameters of the square wave jerks in data obtained from a potential patient against the corresponding parameters of a healthy population.

9. The method as in claim 8 further comprising controlling a processor to define the opposite direction of the saccadic movements in the pairs as 180 degrees, plus or minus 80 degrees.

10. The method as in claim 9 further comprising controlling a processor to define the magnitude of each saccadic movements in the pairs comparable as the dissimilarity index is in the range $\pm 100\%$.

11. An apparatus that detects and characterizes eye movements of a subject, for the differential diagnosis of a patient with progressive supranuclear palsy (PSP) from a patient with Parkinson's disease (PD) comprising:

    an eye movement processing apparatus that identifies a plurality of square wave jerks within a sequence of repetitive eye movements during gaze fixation, each of the plurality of square wave jerks defined by a first horizontal saccadic movement that moves the eye away from a fixation target followed by a corrective saccadic movement towards the target shortly thereafter;
    comprising at least one processor configured to:

       measure vertical components associated with the plurality of square wave jerks;
       measure a saccade rate of the plurality of square wave jerks;
       compare the vertical components with a threshold value;

compare the saccade rate with a threshold value; and
differentially identify progressive supranuclear palsy from Parkinson's disease based upon the comparison of the vertical component with the threshold value and the saccade rate with the threshold value.

12. The apparatus as in claim 11, wherein the eye movements identified by the eye movement processing apparatus further comprises a sequence of saccadic movements.

13. The apparatus as in claim 12, wherein the eye movements identified by the eye movement processing apparatus further comprises pairs of consecutive saccadic movements of the sequence.

14. The apparatus as in claim 13, wherein the eye movement processing apparatus determines whether each saccadic movement of each identified pair is opposite the direction of the other saccadic movement and, if not, then discards the pair.

15. The apparatus as in claim 14, wherein the eye movement processing apparatus determines whether a magnitude of each saccadic movement of each identified pair is comparable and, if not, then discards the pair.

16. The apparatus as in claim 15, wherein the eye movement processing apparatus determines whether the pair of saccadic movements of each identified pair are temporally related by a predetermined time period and, if not, then discarding the pair.

17. The apparatus as in claim 16, wherein the eye movement processing apparatus collects any remaining pairs of saccadic movements as square wave jerks.

18. The apparatus as in claim 17, wherein the eye movement processing apparatus compares a set of parameters of the eye movements in a potential patient against the corresponding parameters of a healthy population.

19. The apparatus as in claim 18, wherein the eye movement processing apparatus defines the opposite direction of the saccadic movements in the pairs as 180 degrees, plus or minus 80 degrees.

**Patentansprüche**

1. Verfahren zur Steuerung einer Vorrichtung, die zwischen einem Patienten mit progressiver supranukleärer Blickparese (PSP) und einem Patienten mit Parkinson-Krankheit (PD) unterscheidet, wobei das Verfahren die folgenden Schritte umfasst:

a) Steuern eines Prozessors zur Bestimmung der Sakkadenrate aus zuvor von einem Patienten gewonnenen Daten und der vertikalen Komponente der mit einer Vielzahl von Rechteckzuckungen verbundenen Daten, die zuvor von einem Patienten erhalten wurden,
b) Steuern eines Prozessors zum Vergleichen der Sakkadenrate mit einem Schwellenwert und der vertikalen Komponente mit einem Schwellenwert,
c) Steuern eines Prozessors, um auf der Grundlage des Vergleichs der Sakkadenrate und der vertikalen Komponenten mit den Schwellenwerten das Vorhandensein von PSP oder PD bei dem Patienten festzustellen, wobei eine Sakkadenrate, die sich statistisch von dem Schwellenwert unterscheidet, und eine vertikale Komponente, die sich statistisch von dem Schwellenwert unterscheidet, den zu untersuchenden Patienten als einen solchen mit PSP identifiziert, während eine Sakkadenrate, die sich statistisch von dem Schwellenwert unterscheidet, und eine vertikale Komponente, die sich nicht statistisch von dem Schwellenwert unterscheidet, den Patienten als einen solchen mit PD identifiziert,

wobei die zuvor von dem Patienten erhaltenen Daten eine Aufzeichnung einer Vielzahl von zumindest teilweise repetitiven Augenbewegungen während der Blickfixierung umfassen, die im Laufe der Zeit Rechteckzuckungen definieren, wobei jede Rechteckzuckung durch eine erste horizontale Sakkadenbewegung definiert ist, die das Auge von einem Fixationsziel wegbewegt, auf die kurz darauf eine korrigierende Sakkadenbewegung zum Ziel hin folgt.

2. Verfahren nach Anspruch 1, wobei die Sequenz der Sakkadenbewegungen zuvor mit Hilfe eines Prozessors erfasst wurde.

**3.** Verfahren nach Anspruch 2, ferner das Steuern eines Prozessors zur Erkennung von Paaren aufeinanderfolgender Sakkadenbewegungen der Sequenz umfassend.

**4.** Verfahren nach Anspruch 3, ferner umfassend das Steuern eines Prozessors zur Feststellung, ob jede Sakkadenbewegung jedes identifizierten Paares der Richtung der anderen Sakkadenbewegung entgegengesetzt ist, und falls nicht, das Paar zu verwerfen.

**5.** Verfahren nach Anspruch 4, ferner umfassend das Steuern eines Prozessors zur Feststellung, ob die Größe jeder Sakkadenbewegung jedes identifizierten Paares vergleichbar ist, und falls nicht, das Paar zu verwerfen.

**6.** Verfahren nach Anspruch 5, ferner umfassend das Steuern eines Prozessors zur Feststellung, ob die beiden Sakkadenbewegungen eines jeden identifizierten Paares zeitlich durch eine vorbestimmte Zeitspanne zusammenhängen, und falls nicht, das Paar zu verwerfen.

**7.** Verfahren nach Anspruch 6, wobei alle verbleibenden Paare von Sakkadenbewegungen zuvor mit einem Prozessor als Rechteckzuckungen erfasst wurden.

**8.** Das Verfahren nach Anspruch 7, ferner umfassend das Steuern eines Prozessors, um einen Satz von Parametern der Rechteckzuckungen in den Daten, die von einem potenziellen Patienten erhalten wurden, mit den entsprechenden Parametern einer gesunden Population zu vergleichen.

**9.** Verfahren nach Anspruch 8, ferner umfassend das Steuern eines Prozessors, um die entgegengesetzte Richtung der sakkadischen Bewegungen in den Paaren als 180 Grad plus oder minus 80 Grad zu definieren.

**10.** Verfahren nach Anspruch 9, ferner umfassend das Steuern eines Prozessors, um die Größe aller Sakkadenbewegungen in den Paaren als vergleichbar zu definieren, wenn der Unähnlichkeitsindex im Bereich von $\pm 100\%$ liegt.

**11.** Vorrichtung, die Augenbewegungen eines Subjekts detektiert und charakterisiert, für die Differentialdiagnose eines Patienten mit progressiver supranukleärer Blickparese (PSP) von einem Patienten mit der Parkinson-Krankheit (PD), umfassend:

eine Augenbewegungs-Verarbeitungsvorrichtung, die eine Vielzahl von Rechteckzuckungen innerhalb einer Sequenz repetitiver Augenbewegungen während der Blickfixation identifiziert, wobei jede der Vielzahl von Rechteckzuckungen durch eine erste horizontale Sakkadenbewegung definiert ist, die das Auge von einem Fixationsziel wegbewegt, kurz danach gefolgt von einer korrigierenden Sakkadenbewegung zum Ziel hin; umfassend mindestens einen Prozessor, der eingerichtet ist, um:

- vertikale Komponenten zu messen, die zu der Vielzahl von Rechteckzuckungen gehören,
- die Sakkadenrate der Vielzahl von Rechteckzuckungen zu messen,
- die vertikalen Komponenten mit einem Schwellenwert zu vergleichen,
- die Sakkadenrate mit einem Schwellenwert zu vergleichen und
- auf der Grundlage des Vergleichs der vertikalen Komponenten mit dem Schwellenwert und der Sakkadenrate mit dem Schwellenwert progressive supranukleäre Blickparese von Parkinson-Krankheit differenzierend zu erkennen.

**12.** Vorrichtung nach Anspruch 11, wobei die von der Augenbewegungs-Verarbeitungsvorrichtung identifizierten Augenbewegungen ferner eine Sequenz von Sakkadenbewegungen umfassen.

**13.** Vorrichtung nach Anspruch 12, wobei die von der Augenbewegungs-Verarbeitungsvorrichtung identifizierten Augenbewegungen ferner Paare von aufeinanderfolgenden Sakkadenbewegungen der Sequenz umfassen.

**14.** Vorrichtung nach Anspruch 13, wobei die Augenbewegungs-Verarbeitungsvorrichtung feststellt, ob jede Sakkadenbewegung jedes identifizierten Paares der Richtung der anderen Sakkadenbewegungen entgegengesetzt ist, und falls nicht, das Paar verwirft.

**15.** Vorrichtung nach Anspruch 14, wobei die Augenbewegungs-Verarbeitungsvorrichtung feststellt, ob die Größe jeder Sakkadenbewegung jedes identifizierten Paares vergleichbar ist, und falls nicht, das Paar verwirft.

**16.** Vorrichtung nach Anspruch 15, wobei die Augenbewegungs-Verarbeitungsvorrichtung feststellt, ob beide Sakkadenbewegungen jedes identifizierten Paares zeitlich um eine vorbestimmte Zeitspanne zusammenhängen, und falls nicht, das Paar verwirft.

**17.** Vorrichtung nach Anspruch 16, wobei die Augenbewegungs-Verarbeitungsvorrichtung alle verbleibenden Paare von Sakkadenbewegungen als Rechteckzuckungen erfasst.

**18.** Vorrichtung nach Anspruch 17, wobei die Augenbewegungs-Verarbeitungsvorrichtung einen Satz von Parametern der Augenbewegungen eines potentiellen Patienten mit den entsprechenden Parametern einer gesunden Population vergleicht.

**19.** Vorrichtung nach Anspruch 18, wobei die Augenbewegungs-Verarbeitungsvorrichtung die entgegengesetzte Richtung der Sakkadenbewegungen in den Paaren als 180 Grad plus oder minus 80 Grad definiert.

**Revendications**

**1.** Procédé de commande d'un appareil qui différencie un patient atteint de paralysie supranucléaire progressive (PSP) d'un patient atteint de la maladie de Parkinson (PD), le procédé comprenant les étapes suivantes :

   a) commander un processeur pour déterminer une vitesse de saccade de données précédemment obtenues d'un patient et une composante verticale des données précédemment obtenues d'un patient, associées à un ensemble de secousses d'ondes carrées ;
   b) commander un processeur pour comparer la vitesse de saccade à une valeur seuil et la composante verticale à une valeur seuil ;
   c) commander un processeur pour identifier la présence d'une paralysie PSP ou d'une maladie PD chez le patient sur la base de la comparaison de la vitesse de saccade et des composantes verticales avec les valeurs de seuil, une vitesse de saccade statistiquement différente de la valeur de seuil et une composante verticale statistiquement différente de la valeur de seuil identifiant le patient comme étant diagnostiqué comme ayant une paralysie PSP, tandis qu'une vitesse de saccade statistiquement différente de la valeur de seuil et une composante verticale non statistiquement différente de la valeur de seuil identifient le patient comme ayant une maladie PD,

les données précédemment obtenues du patient comprenant un enregistrement d'un ensemble de mouvements oculaires au moins partiellement répétitifs pendant un regard fixé qui, au fil du temps, définissent des saccades d'ondes carrées, chaque saccade d'onde carrée étant définie par un premier mouvement saccadique horizontal éloignant l'œil d'une cible de fixation et suivi peu après d'un mouvement saccadique correctif vers la cible.

**2.** Procédé selon la revendication 1,
dans lequel
la séquence de mouvements saccadés a été préalablement acquise en utilisant un processeur.

**3.** Procédé selon la revendication 2,
consistant en outre à commander un processeur pour identifier des paires de mouvements saccadés consécutifs de la séquence.

**4.** Procédé selon la revendication 3,
consistant en outre à commander un processeur pour déterminer si chaque mouvement saccadique de chaque paire identifiée est opposé à la direction de l'autre mouvement saccadique et, si ce n'est pas le cas, éliminer cette paire.

**5.** Procédé selon la revendication 4,
comprenant en outre la commande d'un processeur pour déterminer si l'amplitude de chaque mouvement saccadique de chaque paire identifiée est comparable et, si ce n'est pas le cas, éliminer cette paire.

**6.** Procédé selon la revendication 5,
consistant en outre à commander un processeur pour déterminer si la paire de mouvements saccadiques de chaque paire identifié est liée temporellement par une période de temps prédéterminée et, si ce n'est pas le cas, éliminer cette paire.

7. Procédé selon la revendication 6,
dans lequel
toutes les paires restantes de mouvements saccadés ont été précédemment acquises avec un processeur en tant que saccades d'ondes carrées.

8. Procédé selon la revendication 7,
consistant en outre à commander un processeur pour comparer un ensemble de paramètres des saccades d'ondes carrées dans des données obtenues à partir d'un patient potentiel par rapport aux paramètres correspondants d'une population saine.

9. Procédé selon la revendication 8,
consistant en outre à commander un processeur pour définir la direction opposée des mouvements saccadés dans les paires pour 180 degrés, plus ou moins 80 degrés.

10. Procédé selon la revendication 9,
consistant en outre à commander un processeur pour définir l'amplitude de chaque mouvement saccadique dans les paires comparables lorsque l'indice de dissimilarité est dans la plage ±100 %.

11. Appareil détectant et caractérisant les mouvements oculaires d'un sujet, pour le diagnostic différentiel d'un patient atteint de paralysie supranucléaire progressive (PSP) par rapport à un patient atteint de la maladie de Parkinson (PD) comprenant :

un appareil de traitement des mouvements oculaires qui identifie un ensemble de saccades d'ondes carrées dans une séquence de mouvements oculaires répétitifs pendant la fixation du regard, chacun des ensembles de saccades d'ondes carrées étant défini par un premier mouvement saccadique horizontal qui éloigne l'œil d'une cible de fixation suivi peu après d'un mouvement saccadique correctif vers la cible ;
comprenant au moins un processeur configuré pour :

mesurer les composantes verticales associées à l'ensemble de secousses d'ondes carrées ;
mesurer une vitesse de saccade de l'ensemble de secousses d'ondes carrées ;
comparer les composantes verticales à une valeur de seuil ;
comparer la vitesse de saccade à une valeur de seuil ; et
identifier de manière différentielle, la paralysie supranucléaire progressive de la maladie de Parkinson par la comparaison de la composante verticale à la valeur de seuil et de la vitesse de saccade, à la valeur de seuil.

12. Appareil selon la revendication 11,
dans lequel
les mouvements oculaires identifiés par l'appareil de traitement des mouvements oculaires comprennent en outre une séquence de mouvements saccadés.

13. Appareil selon la revendication 12,
dans lequel
les mouvements oculaires identifiés par l'appareil de traitement des mouvements oculaires comprennent en outre des paires de mouvements saccadés consécutifs de la séquence.

14. Appareil selon la revendication 13,
dans lequel
l'appareil de traitement des mouvements oculaires détermine si chaque mouvement saccadique de chaque paire identifiée est opposé à la direction de l'autre mouvement saccadique et, si ce n'est pas le cas, il élimine la paire.

15. Appareil selon la revendication 14,
dans lequel
l'appareil de traitement des mouvements oculaires détermine si l'amplitude de chaque mouvement saccadé de chaque paire identifiée est comparable et, si ce n'est pas le cas, il élimine la paire.

16. Appareil selon la revendication 15,

dans lequel

l'appareil de traitement des mouvements oculaires détermine si la paire de mouvements saccadiques de chaque paire identifiée est liée temporellement par une période de temps prédéterminée et, si ce n'est pas le cas, il élimine la paire.

**17.** Appareil selon la revendication 16,

dans lequel

l'appareil de traitement des mouvements oculaires collecte toutes les paires restantes de mouvements saccadiques sous forme de secousses d'ondes carrées.

**18.** Appareil selon la revendication 17,

dans lequel

l'appareil de traitement des mouvements oculaires compare un ensemble de paramètres des mouvements oculaires chez un patient potentiel aux paramètres correspondants d'une population saine.

**19.** Appareil selon la revendication 18,

dans lequel

l'appareil de traitement des mouvements oculaires définit la direction opposée des mouvements saccadiques dans les paires pour 180 degrés, plus ou moins 80 degrés.

## Fig. 1

## Fig. 2

HORIZONTAL EYE POSITION
(DEGREES OF VISUAL ANGLE)

PSP PATIENTS

HEALTHY CONTROLS

VIDEO TRACKING

TIME(S)

EYE COIL TRACKING

Fig. 3

RECORD EYE MOVEMENTS —200

DETECT SMALL SACCADES —202

GET FIRST PAIR OF CONSECUTIVE SACCADES —204

206— MEASURE DIRECTION DIFFERENCE, RELATIVE MAGNITUDE DIFFERENCE AND INTER-SACCADIC INTERVAL

208— GET NEXT PAIR OF CONSECUTIVE SACCADES

210— VALID DIRECTION DIFFERENCE? — NO / YES

212— VALID RELATIVE MAGNITUDE DIFFERENCE? — NO / YES

214— VALID INTER-SACCADIC INTERVAL? — NO / YES

216— SAVE SACCADE PAIR AS POTENTIAL SWJ

218— MORE SACCADE PAIRS? — YES / NO

220— FIND SEQUENCES OF POTENTIAL SWJ SHARING SACCADES

222— GET FIRST SEQUENCE

224— GET NEXT SEQUENCE

226— SELECT THE SWJS IN THE ODD POSITIONS IN THE SEQUENCES

228— EVEN NUMBER OF SWJS IN THE SEQUENCE? — NO / YES

230— SELECT THE SWJS IN THE EVEN OR ODD POSITIONS IN THE SEQUENCES ACCORDING TO THE INTER-SACCADIC INTERVALS

232— MORE SEQUENCES? — YES / NO

234— FINISH

Fig. 4A

1ST SACCADE IN RED; ACCEPTABLE DIRECTIONS
OF 2ND SACCADE IN BLUE

Fig. 4B

AVG. MAGNITUDE OF 1ST
AND 2ND SACCADE

Fig. 4C

TIME BETWEEN 1ST
AND 2ND SACCADE

Fig. 4D

DIFFERENCE IN DIRECTION BETWEEN
THE 1ST AND THE 2ND SACCADE (DEG.)

Fig. 4E

RELATIVE MAGNITUDE DIFFERENCE
OF 1ST AND THE 2ND SACCADE (%)

Fig. 4F

INTER-SACCADIC INTERVAL(MS)

Fig. 5A

Fig. 5B

Fig. 5C

Fig. 5D

Fig. 5E

Fig. 5F

Fig. 5G

Fig. 5H

Fig. 6

# Fig. 7

Fig. 7A

Fig. 7B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 12740008 A

**Non-patent literature cited in the description**

- **CHEN, A.L. et al.** *Frontiers in Neurology,* 2010, vol. 1 (147), 1-19
- **MARTINEZ-CONDE ; MACKNIK, HUBEI.** *Nature Neuroscience,* 2000, vol. 3, 251-258
- **ENGBERT, KLIEGL.** *Vision Res,* 2003, vol. 43, 1035-1045